# EUROPÄISCHE PATENTSCHRIFT

(11) **EP 3 307 895 B1**
(45) Veröffentlichungstag und Bekanntmachung des Hinweises auf die Patenterteilung: **28.07.2021**
(21) Anmeldenummer: 16728662.4
(22) Anmeldetag: 06.06.2016
(51) Int. Cl.: C12P 5/02, C12P 7/22, C12P 7/40, C12P 7/52, C12P 17/10

(54) **VERFAHREN ZUR GLEICHZEITIGEN HERSTELLUNG VON BIOGAS UND MINDESTENS EINER ORGANISCHEN VERBINDUNG**
METHOD FOR SIMULTANEOUS PRODUCTION OF BIOGAS AND AT LEAST ONE ORGANIC COMPOUND
PROCÉDÉ POUR LA PRODUCTION SIMULTANÉE DE BIOGAZ ET D'AU MOINS UN COMPOSÉ ORGANIQUE

(30) Priorität: 15.06.2015 DE 102015210871
(43) Veröffentlichungstag der Anmeldung: 18.04.2018
(73) Patentinhaber: Verbio Vereinigte Bioenergie AG, 04109 Leipzig (DE)
(72) Erfinder: LÜDTKE, Oliver, 04416 Markkleeberg (DE); SCHLIMBACH, Michael, 06120 Halle (DE); FICHTER, Enrico, 04315 Leipzig (DE)
(74) Vertreter: Gulde & Partner
(86) Internationale Anmeldenummer: PCT/EP2016/062736
(87) Internationale Veröffentlichungsnummer: WO 2016/202616

(56) Entgegenhaltungen:
- WO-A1-2007/014717
- WO-A1-2014/072756
- WO-A2-98/04729
- WO-A2-2009/047275

## Beschreibung

### Hintergrund der Erfindung

### Technisches Gebiet

Die Erfindung betrifft ein Verfahren zur stofflichen Nutzung heterogenen organischen Substrats durch einen anaeroben Abbauprozess, wobei das organische Substrat zu mindestens einem organischen Zielprodukt und Biogas abgebaut wird.

### Beschreibung des Standes der Technik

Der Abbau von organischem Substrat durch mikrobielle anaerobe Prozesse, auch Fermentationen genannt, ist bekannt. Üblicherweise werden solche Fermentationen mit dem Ziel der Gewinnung eines werthaltigen Produkts aus einem vergleichsweise preiswerten Substrat durchgeführt. Bei dem eingesetzten Substrat handelt es sich um eine organische Substanz bzw. ein Gemisch verschiedener organischer Substanzen. Je nach Substrat und gewünschtem Produkt sind bestimmte Mikroorganismen oder Gruppen von Mikroorganismen für den Fermentationsprozess geeignet. Für die erfolgreiche Durchführung einer Fermentation sind neben der Substratversorgung auch die Versorgung mit Nährstoffen sowie die Einhaltung günstiger Prozessparameter wie zum Beispiel Temperatur und Druck sicherzustellen.

Die Bildung von Biogas bzw. Biomethan aus organischem Substrat ist ein Beispiel für einen technisch genutzten mikrobiellen anaeroben Prozess. Die Biogasproduktion hat in den letzten Jahren zunehmend an Bedeutung gewonnen. Während zunächst die anaerobe Behandlung von Klärschlamm primär mit dem Ziel einer Schlammreduktion unter Bildung von Biogas im Zentrum des Interesses stand, lag der Fokus in jüngerer Vergangenheit auf der Gewinnung von Biogas aus landwirtschaftlicher Anbaubiomasse wie zum Beispiel Ganzpflanzensilage, teilweise auch kombiniert mit der Vergärung von Gülle oder anderen Exkrementen der Tierhaltung. Für eine hohe Rentabilität eines solchen Biogasprozesses ist eine möglichst hohe Biogasausbeute bezogen auf das eingesetzte organische Substrat erforderlich. Es gibt eine Reihe von technischen Vorschlägen, welche dieses Ziel verfolgen. Stand der Technik zur Erreichung hoher Biogasausbeuten sind eine moderate, gleichbleibende Substratbelastung und eine ausreichende Versorgung mit Makro- und Mikronährstoffen sowie die Realisierung einer möglichst langen Verweilzeit des Substrats im Fermentationsprozess. Dabei ist im Hinblick auf die Wirtschaftlichkeit einer Anlage ein Kompromiss zwischen hoher Raumbelastung und langer Verweilzeit und damit verbundener hoher Biogasausbeute zu finden. Üblicherweise besteht der Kompromiss darin, die Anlagenleistung soweit zu steigern, bis die Prozessgüte messbar sinkt oder die Prozessstabilität abnimmt. Die Bewertung der Prozessgüte bzw. der Prozessstabilität erfolgt anhand der Biogasausbeute bzw. aus der Analyse des Fermenterinhalts auf das Vorhandensein von flüchtigen organischen Säuren (FOS). Diese FOS (zum Beispiel Essigsäure, Propionsäure, Buttersäure und andere Carbonsäuren) sind Zwischenprodukte des anaeroben Substratabbaus. Ihre Anreicherung im Fermenterinhalt bedeutet in Bezug auf die Methanbildung einen unvollständigen Substratabbau. Daher wird nach aktueller Lehre bei der Biogasproduktion eine niedrige Konzentration der FOS angestrebt. Die Konzentration der FOS kann quantitativ zum Beispiel durch chromatographische Analysen oder halbquantitativ als Summenparameter (FOS-Wert bzw. FOS/TAC-Wert) durch Titrationsverfahren bestimmt werden.

Ebenfalls existent sind Konzepte zur Effizienzsteigerung von Biogasanlagen durch mehrstufige Prozesse. Dem Fachmann bekannt und in der Praxis üblich sind Anlagen mit einer räumlichen Trennung in Hauptvergärung und Nachvergärung, welche ihrerseits wieder mehrstufig ausgeführt sein können. Teilweise wird die Nachvergärung auch gleichzeitig als Gärrestlager genutzt. Dabei erfolgt die Substratzufuhr in der Regel nur in die Hauptvergärung. Dadurch wird eine Mindestverweilzeit des frischen Substrats sichergestellt. Die einzelnen Stufen unterscheiden sich hinsichtlich der Prozessbedingungen und ihrer mikrobiologischen Zusammensetzung nicht wesentlich voneinander. Es laufen alle Abbauprozesse nebeneinander ab.

Ein solcher Prozess wird beispielsweise in EP2419516B1 beschrieben. Bei diesem Verfahren erfolgt in der ersten Stufe eine Vergärung des feststoffhaltigen Substrats unter geringer Akkumulation von FOS bis maximal 4 g/l, vorzugsweise bis 2 g/l. In der zweiten Stufe wird das teilverflüssigte und teilumgesetzte Substrat vollständig ausgegoren. Durch Trennung in zwei Stufen und unterschiedliche Durchmischungen in den beiden Stufen soll die Ausbeute des Gesamtprozesses verbessert werden. Die Einhaltung eines niedrigen FOS-Gehalts in der ersten Stufe soll der Verhinderung einer Inhibierung der Methanbildung dienen und wird durch Rückführung von Ablauf aus der zweiten Stufe erreicht.

Eine räumliche Trennung der mikrobiologischen Abbauprozesse einer Biogasanlage mit dem Ziel der Steigerung der Anlageneffizienz ist dem Fachmann ebenfalls bekannt. Derartige Prozesse werden zum Beispiel in DE19937876C2 und DE102011106772A1 vorgeschlagen.

DE19937876C2 offenbart ein zweistufiges Verfahren, welches aus einer gesteuerten Hydrolyse und einer räumlich getrennten Methanbildung besteht. Durch gezielte Einstellung des pH-Werts und des Redoxpotenzials werden in der Hydrolysestufe die Säurebildung gefördert und gleichzeitig die Methanbildung unterdrückt. In der zweiten Stufe werden die Prozessbedingungen in einem für die methanogenen Mikroorganismen günstigen Bereich eingestellt. Nachteilig bei diesem Prozess ist die Regelung des pH und Redox in der Hydrolyse durch Einblasen von Gasen, insbesondere von Luft, da der Eintrag von Sauerstoff zwangsläufig zu einem anteilig aeroben Abbau und damit einer Verminderung der Ausbeute und zu Problemen der weiteren Gasnutzung führt.

DE102011106772A1 lehrt ein zweistufiges Verfahren zur Erzeugung von Methangas aus einem Feststoffe und Flüssigkeit enthaltenden organischen Reststoff, bestehend aus Hydrolyse und Methanbildung. Es wird vorgeschlagen die Hydrolyse derart zu betreiben, dass in dieser kein Methan gebildet wird, damit aus dieser Feststoffe ohne Methanbildungspotenzial abgetrennt werden können. Die nach der Feststoffabtrennung in der Flüssigkeit enthaltenen organischen Säuren werden in der zweiten Stufe vollständig zu Methangas umgesetzt.

Darüber hinaus ist auch die Gewinnung der in einer Hydrolyse gebildeten organischen Säuren bekannt. Beispielsweise ist in WO2014072756A1 ein Prozess zum mikrobiellen Abbau von organischem Substrat zur Rückgewinnung von Wertstoffen und Nährstoffen vorgeschlagen. Wesentliches Merkmal ist die mikrobielle Hydrolyse und Versäuerung des Substrates zur Gewinnung eines organische Säuren enthaltenden Permeats. Die Hydrolyse und Versäuerung des Substrats wird durch Einstellung des pH, des Redoxwertes oder auch der Temperatur sowie sehr kurzer Verweilzeit gefördert. Das organische Säuren enthaltene Permeat kann in weiteren Prozessen Verwendung finden.

In EP0918876B1 ist ebenfalls ein Prozess zur Gewinnung von organischen Säuren bzw. Salzen von organischen Säuren durch anaerobe Fermentation aus Biomasse offenbart. Es ist dargestellt, dass ein pH unter 5,8 die Methanbildung effektiv hemmt. Darüber hinaus kann durch Dosierung von Inhibitoren die Methanbildung unterdrückt werden. Das beim anaeroben Abbau zu Säuren entstehende Gas besteht aus Kohlendioxid, Wasserstoff und gegebenenfalls auch aus Methan. Es ist erwähnt, dass für eine hohe Ausbeute an Säuren möglichst wenig Gas gebildet werden muss.

WO-A-2009/047275 offenbart ein Verfahren zur Erzeugung von Methangas und eines anderen Produktes in einer Co-Kultur in einem Reaktionsraum, d.h. einem Reaktor ohne räumliche Trennung mit Glycerin als Substrat.

Die derzeit bekannten Verfahren zur Vergärung von organischem Substrat haben zur Zielstellung entweder die Erzeugung von Biogas bzw. Biomethan oder fokussieren auf die Erzeugung von organischen Säuren. Während bei der Erzeugung von Biogas bzw. Biomethan organische Säuren als ausbeutemindernd zu betrachten sind und deren Abbau bewusst gefördert wird, ist bei der Erzeugung von Wertstoffen in einer Hydrolyse der weitere Abbau zu Biogas bzw. Methan unerwünscht und wird gezielt unterdrückt.

Zwar existieren Konzepte zur Kombination einer separaten Hydrolyse und nachgeschalteter Methangärung. Jedoch zielen diese allein auf eine Effizienzsteigerung der Biogas- bzw. Biomethanausbeute ab. Zudem ergeben sich durch die dabei realisierte räumliche Trennung der mikrobiellen Abbaustufen Nachteile in Bezug auf die Gasbildung, da auch in der Hydrolysestufe Gas freigesetzt wird, welches neben Kohlendioxid auch relevante Mengen Wasserstoff und Methan enthält. Darüber hinaus entsteht insbesondere bei proteinreichen Substraten in der Hydrolyse und Versäuerung auch Schwefelwasserstoff. Der Gasstrom aus der Hydrolyse muss in jedem Fall einer Reinigung unterzogen werden. Eine ökonomische Nutzung des aus einer reinen Hydrolyse und/oder Versäuerung gewonnenen Gasstroms ist schwierig.

Es sind keine Verfahren bekannt, welche ohne räumliche Trennung der verschiedenen Abbaustufen die Erzeugung eines Zielprodukts und gleichzeitig von Biogas bzw. Methan in einem Reaktionsraum realisieren.

### Aufgabe und Lösung

Es ist daher Aufgabe der Erfindung, heterogenes organisches Substrat, insbesondere auch organische Reststoffe, durch einen effizienten und kostengünstigen anaeroben Abbauprozess zu mindestens einem organischen Zielprodukt und Biogas umzuwandeln und so eine signifikante Wertschöpfung zu erzielen.

Die Aufgabe wird durch ein Verfahren zur Gewinnung mindestens eines organischen Zielprodukts und von Biogas gelöst, welches folgende Schritte umfasst:
a) heterogenes organisches Substrat einem anaeroben Fermentationsprozess zugeführt wird;
b) bei diesem anaeroben Fermentationsprozess eine Hydrolyse, Versäuerung und Methanisierung durch eine Mischkultur aus Bakterien und Archaeen in einem Reaktionsraum durchgeführt werden;
c) eine partielle Unterdrückung der anaeroben Fermentation durch die hydraulische Verweilzeit des anaeroben Fermentationsprozesses und/oder die Substratzufuhr und/oder die Temperatur des anaeroben Fermentationsprozesses und/oder die Limitierung mindestens eines Nährstoffs im anaeroben Fermentationsprozess und/oder die Konzentration eines Hemmstoffes im anaeroben Fermentationsprozess gesteuert wird, wodurch ein Teil des organischen Substrats nicht vollständig zu Biogas bzw. Methan abgebaut wird;
d) mindestens ein Zielprodukt als organisches Stoffwechselprodukt einer Mischkultur aus Bakterien im Fermentationsprozess durch den gezielten unvollständigen Abbau des organischen Substrats angereichert wird;
e) das im anaeroben Fermentationsprozess durch die Archaeen gebildete Biogas einen Anteil von Wasserstoff von kleiner 5% aufweist und zur weiteren Verwendung gewonnen wird; und
f) mindestens ein organisches Zielprodukt zur weiteren Verwendung aus dem anaeroben Fermentationsprozess gewonnen wird.

Zur Lösung dieser Aufgabe stellt die Erfindung ein Verfahren gemäß Anspruch 1 bereit. Vorteilhafte Weiterbildungen sind in den abhängigen Ansprüchen genannt.

In einer bevorzugten Ausführungsform ist das erfindungsgemäße Verfahren so ausgestaltet, dass
der Anteil an Wasserstoff des beim anaeroben Fermentationsprozess gebildeten Biogases kleiner 2%, besonders bevorzugt kleiner 1% ist.

In einer weiteren bevorzugten Ausführungsform ist das erfindungsgemäße Verfahren so ausgestaltet, dass aus dem anaeroben Fermentationsprozesses mindestens zwei Zielprodukte separat gewonnen werden.

In einer weiteren bevorzugten Ausführungsform ist das erfindungsgemäße Verfahren so ausgestaltet, dass eines der Zielprodukte eine organische Säure enthält.

In einer weiteren bevorzugten Ausführungsform ist das erfindungsgemäße Verfahren so ausgestaltet, dass eines der Zielprodukte eine aromatische Verbindung enthält.

In einer weiteren bevorzugten Ausführungsform ist das erfindungsgemäße Verfahren so ausgestaltet, dass ein bei der Gewinnung eines Zielprodukts anfallender Stoffstrom wieder einem anaeroben Fermentationsprozess zugeführt wird.

In einer weiteren bevorzugten Ausführungsform ist das erfindungsgemäße Verfahren so ausgestaltet, dass die Anreicherung mindestens eines Zielprodukts durch die hydraulische Verweilzeit des anaeroben Fermentationsprozesses gesteuert wird.

In einer weiteren bevorzugten Ausführungsform ist das erfindungsgemäße Verfahren so ausgestaltet, dass die Anreicherung mindestens eines Zielprodukts durch die Substratzufuhr des anaeroben Fermentationsprozesses gesteuert wird.

In einer weiteren bevorzugten Ausführungsform ist das erfindungsgemäße Verfahren so ausgestaltet, dass die Anreicherung mindestens eines Zielprodukts durch die Temperatur des anaeroben Fermentationsprozesses gesteuert wird.

In einer weiteren bevorzugten Ausführungsform ist das erfindungsgemäße Verfahren so ausgestaltet, dass die Anreicherung mindestens eines Zielprodukts durch Limitierung mindestens eines Nährstoffs im anaeroben Fermentationsprozess gesteuert wird.

In einer weiteren bevorzugten Ausführungsform ist das erfindungsgemäße Verfahren so ausgestaltet, dass die Anreicherung mindestens eines Zielprodukts durch Limitierung mindestens eines Nährstoffs bestehend aus den Elementen Bor, Eisen, Kalium, Kobalt, Kupfer, Magnesium, Mangan, Molybdän, Natrium, Nickel, Phosphor, Schwefel, Selen, Stickstoff, Wolfram oder Zink im anaeroben Fermentationsprozess gesteuert wird.

In einer weiteren bevorzugten Ausführungsform ist das erfindungsgemäße Verfahren so ausgestaltet, dass die Anreicherung mindestens eines Zielprodukts durch die Konzentration eines Hemmstoffes im anaeroben Fermentationsprozess gesteuert wird.

In einer weiteren bevorzugten Ausführungsform ist das erfindungsgemäße Verfahren so ausgestaltet, dass die Anreicherung mindestens eines Zielprodukts durch die Konzentration des Sulfidgehalts im anaeroben Fermentationsprozess gesteuert wird.

In einer weiteren bevorzugten Ausführungsform ist das erfindungsgemäße Verfahren so ausgestaltet, dass die Anreicherung mindestens eines Zielprodukts durch die Konzentration des Ammoniumgehalts im anaeroben Fermentationsprozess gesteuert wird.

In einer weiteren bevorzugten Ausführungsform ist das erfindungsgemäße Verfahren so ausgestaltet, dass die Anreicherung mindestens eines Zielprodukts durch die Konzentration des Ammoniumgehalts von mindestens 4.000 mg/l, bevorzugt mindestens 5.000 mg/l, besonders bevorzugt mindestens 6.000 mg/l und ganz besonders bevorzugt mindestens 7.000 mg/l im Fermentationsmedium gesteuert wird.

In einer weiteren bevorzugten Ausführungsform ist das erfindungsgemäße Verfahren so ausgestaltet, dass mindestens ein Zielprodukt durch Strippung gewonnen wird.

In einer weiteren bevorzugten Ausführungsform ist das erfindungsgemäße Verfahren so ausgestaltet, dass mindestens ein Zielprodukt durch mindestens ein thermisches Trennverfahren wie zum Beispiel durch Destillation gewonnen wird.

In einer weiteren bevorzugten Ausführungsform ist das erfindungsgemäße Verfahren so ausgestaltet, dass mindestens ein Zielprodukt durch Extraktion gewonnen wird.

In einer weiteren bevorzugten Ausführungsform ist das erfindungsgemäße Verfahren so ausgestaltet, dass die Gewinnung eines Zielprodukts nach einer Fest-Flüssig-Trennung des Fermentationsmediums erfolgt.

In einer weiteren bevorzugten Ausführungsform ist das erfindungsgemäße Verfahren so ausgestaltet, dass der anaerobe Fermentationsprozess mehrstufig durchgeführt wird.

In einer weiteren bevorzugten Ausführungsform ist das erfindungsgemäße Verfahren so ausgestaltet, dass der anaerobe Fermentationsprozess bei einem pH-Wert größer 6, vorzugsweise größer 7, bevorzugt größer 7,5 realisiert wird.

In einer weiteren bevorzugten Ausführungsform ist das erfindungsgemäße Verfahren so ausgestaltet, dass der anaerobe Fermentationsprozess mindestens quasikontinuierlich durchgeführt wird.

In einer weiteren bevorzugten Ausführungsform ist das erfindungsgemäße Verfahren so ausgestaltet, dass die Gewinnung mindestens eines Zielprodukts simultan mit der Fermentation erfolgt.

In einer weiteren bevorzugten Ausführungsform ist das erfindungsgemäße Verfahren so ausgestaltet, dass die Gewinnung mindestens eines Zielprodukts nach einem ersten Fermentationsprozess erfolgt.

In einer weiteren bevorzugten Ausführungsform ist das erfindungsgemäße Verfahren so ausgestaltet, dass eines der Zielprodukte eine organische Säure enthält.

In einer weiteren bevorzugten Ausführungsform ist das erfindungsgemäße Verfahren so ausgestaltet, dass eines der Zielprodukte eine aromatische Verbindung enthält.

In einer weiteren bevorzugten Ausführungsform ist das erfindungsgemäße Verfahren so ausgestaltet, dass mindestens ein Zielprodukt durch Strippung gewonnen wird.

In einer weiteren bevorzugten Ausführungsform ist das erfindungsgemäße Verfahren so ausgestaltet, dass mindestens ein Zielprodukt durch mindestens ein thermisches Trennverfahren wie zum Beispiel durch Destillation gewonnen wird.

In einer weiteren bevorzugten Ausführungsform ist das erfindungsgemäße Verfahren so ausgestaltet, dass mindestens ein Zielprodukt durch Extraktion gewonnen wird.

In einer weiteren bevorzugten Ausführungsform ist das erfindungsgemäße Verfahren so ausgestaltet, dass der anaerobe Fermentationsprozess mindestens quasikontinuierlich durchgeführt wird.

In einer weiteren bevorzugten Ausführungsform ist das erfindungsgemäße Verfahren so ausgestaltet, dass die Gewinnung mindestens eines Zielprodukts simultan mit der Fermentation erfolgt.

In einer weiteren bevorzugten Ausführungsform ist das erfindungsgemäße Verfahren so ausgestaltet, dass die Gewinnung mindestens eines Zielprodukts nach einem ersten Fermentationsprozess erfolgt.

In einer weiteren bevorzugten Ausführungsform ist das erfindungsgemäße Verfahren so ausgestaltet, dass organisches Substrat in einem einstufigen anaeroben Prozess zu Biogas vergoren wird.

Das erfindungsgemäße Verfahren betrifft insbesondere auch alle Kombinationen der oben beschriebenen, bevorzugten Ausführungsformen.

### Beschreibung der Lösung

Organisches Substrat kann durch einen kontrollierten mikrobiellen Abbau zu Biogas umgesetzt werden. Der mikrobielle Abbau findet im Wesentlichen in drei Stufen statt. Dies sind die Hydrolyse, die Versäuerung und die Methanbildung. Während in den ersten beiden Abbaustufen eine Vielzahl von obligat und fakultativ anaeroben Bakterien den Abbau des organischen Substrats katalysiert, sind für die Methanbildung obligat anaerobe Archaeen verantwortlich. Beim vollständigen Abbau des organischen Substrats, im Wesentlichen bestehend aus den Elementen C, H, O, N, S und P, werden als Endprodukte Kohlenstoffdioxid, Methan und Wasser sowie Ammoniak, Schwefelwasserstoff und anorganisches Phosphat gebildet. Je nach Prozessbedingungen entweichen die Endprodukte als Gase oder liegen im wässrigen Medium gelöst als Ionen vor.

Im Wesentlichen läuft dieser Abbauprozess kaskadenförmig ab. So dienen die in der Hydrolyse aus Makromolekülen gebildeten Substanzen, zumeist langkettige organische Säuren, als Ausgangsstoffe für die Versäuerung. Während der Versäuerung werden die Stoffwechselprodukte aus der Hydrolyse weiter degradiert. Die Versäuerung ist in sich wiederum teilweise kaskadenförmig strukturiert, da größere bzw. längerkettige organische Säuren immer weiter zu niedermolekularen Säuren abgebaut werden. Schließlich dient die gebildete Essigsäure als Ausgangsstoff der acetoclastischen Methanbildung. Während dieser Abbaukaskade, insbesondere in der Versäuerung, werden als Nebenprodukte Kohlendioxid und Wasserstoff gebildet. Der gebildete Wasserstoff wird durch hydrogenotrophe Methanbildung mit einem Teil des Kohlendioxids zu Methan umgesetzt.

Die stofflich wie energetisch sehr enge Kopplung der verschiedenen Abbaustufen wurde vielfach untersucht. Es ist bekannt, dass sich Hydrolyse und Versäuerung von der Methanbildung räumlich trennen lassen. Dies ist gut möglich, da es sich um unterschiedliche Formen von Mikroorganismen mit unterschiedlichen, optimalen Wachstumsbedingungen handelt. So kann durch eine Reihe von Maßnahmen die Methanbildung selektiv unterdrückt werden. Ein niedriger pH-Wert, höhere Redoxwerte zum Beispiel durch Einleiten von Luftsauerstoff, höhere Temperaturen, kurze Verweilzeiten sowie hohe Produktkonzentrationen aus der Versäuerung führen zu einer Unterdrückung und damit Verarmung der Methanbildung. Zusätzlich sind der Fachwelt chemische Verbindungen bekannt, welche bei Dosierung in die Hydrolyse und Säurebildung zu einer selektiven Inhibierung der Methanbildung führen. Beispiele für solche chemischen Verbindungen sind aromatische Verbindungen wie zum Beispiel Phenol, Halogenverbindungen wie zum Beispiel Bromethansulfonsäure bzw. deren Salze oder auch anorganische Verbindungen wie zum Beispiel Ammonium oder Chlorid.

Speziell die Anreicherung von FOS wird oftmals als hemmend für die Methanbildung beschrieben. Die Angaben in der Fachliteratur variieren auch aufgrund der nicht einheitlichen Messmethoden in einem größeren Bereich. Im Allgemeinen werden jedoch FOS über 4 g/l als kritisch bewertet. Ammoniak bzw. Ammonium stellt ein Nebenprodukt bei der Versäuerung von stickstoffhaltigen organischen Verbindungen dar. Hinsichtlich der Ammoniakhemmung gibt es in der Fachliteratur noch größere Schwankungsbreiten. Die allgemeine Lehre besagt, dass höhere Gehalte zu einer Hemmung der Methanbildung führen. Dies gilt ebenso für den in der Versäuerung gebildeten Schwefelwasserstoff.

Eine räumliche Trennung der Abbaustufen wird in der Fachwelt als Möglichkeit zur Effizienzsteigerung gesehen, da in den getrennten Bereichen die jeweils optimalen Prozessbedingungen der beteiligten Mikroorganismen eingestellt werden können. Zumindest im Hinblick auf Wasserstoff stößt dieses Konzept an Grenzen. Der in der Hydrolyse gebildete Wasserstoff steht bei räumlich getrennten Systemen nicht in direktem Kontakt mit den für die Methanbildung verantwortlichen Mikroorganismen und kann demzufolge auch nicht direkt zu Methan umgesetzt werden. Der Wasserstoff ist dann im Hinblick auf die Methanbildung als Verlust zu werten oder muss mit hohem technischen Aufwand in Kontakt mit den Methanbildnern gebracht werden.

Darüber hinaus liegt der Anreiz einer räumlichen Trennung von Hydrolyse und Versäuerung in der Anreicherung von organischen Säuren. Der Fachwelt sind verschiedene Konzepte zur Gewinnung von organischen Säuren aus wässrigen Fermentationsmedien bekannt. Diese Konzepte haben bisher jedoch kaum wirtschaftliche Bedeutung. Organische Säuren werden entweder durch chemische Synthesen aus Ausgangsstoffen fossilen Ursprungs oder durch Spezialfermentationen gewonnen. Bei letzteren kommen in der Regel keine heterogenen organischen Substrate, sondern definierte Medien zum Einsatz.

Zielstellung der vorliegenden Erfindung ist die kostengünstige Gewinnung von organischen Zielprodukten aus organischem Substrat bei gleichzeitiger Bildung von Biogas, insbesondere Biomethan. Dabei ist eine hohe Gesamtausbeute des Substrats im Hinblick auf die Wirtschaftlichkeit von entscheidender Bedeutung.

Überraschenderweise hat sich gezeigt, dass entgegen der allgemeinen Lehre eine signifikante Anreicherung von mindestens einem organischen Zielprodukt in einem einstufigen anaeroben Fermentationsprozess, bei dem die Hydrolyse, Versäuerung und Methanisierung durch eine Mischkultur aus Bakterien und Archaeen ohne räumliche Trennung durchgeführt werden, unter Aufrechterhaltung der Methangärung stabil möglich ist, wenn die anaerobe Fermentation partiell unterdrückt wird. Es wurde zudem gefunden, dass durch eine Reihe von Maßnahmen, insbesondere der Kontrolle der Nährstoffversorgung die partielle Unterdrückung der anaeroben Fermentation und damit die Anreicherung des Zielprodukts beeinflusst werden kann. Darüber hinaus wurde in Versuchen festgestellt, dass man einen im Vergleich zur vollständigen Vergärung gleichbleibend hohen Substratabbau realisieren kann. Dadurch besteht die Möglichkeit, mit günstigem organischem Substrat, wie zum Beispiel organischen Reststoffen, in einem einfachen Fermentationsverfahren werthaltige Zielprodukte sowie Biogas bzw. Methan zu erzeugen.

In einem effizienten kostengünstigen Verfahren wird das zur Verfügung stehende organische Substrat einer anaerob betriebenen Fermentation zugeführt. Bei dem organischen Substrat kann es sich um organische Reststoffe vorgelagerter Prozesse, organische Reststoffe aus dem Nahrungsmittelbereich wie zum Beispiel Essensresten, nachwachsende Rohstoffe insbesondere Stroh oder auch andere beliebige organische Stoffe handeln. Selbstverständlich sind auch Mischungen vorgenannter Stoffe als organische Substrate möglich. Es ist für den Prozess von Vorteil, wenn die Substratzusammensetzung keinen größeren Schwankungen unterliegt. Der Einsatz von Klärschlamm ist eher ungeeignet.

Der Fermenterinhalt besteht aus unvollständig abgebautem Substrat und enthält alle Mikroorganismen, die für einen vollständigen Substratabbau notwendig sind. Das heißt, dass bei diesem anaeroben Fermentationsprozess die Hydrolyse, Versäuerung und Methanisierung durch eine Mischkultur aus Bakterien und Archaeen ohne räumliche Trennung durchgeführt werden. Dabei meint "unvollständig abgebaut", dass ein rechnerischer Teil des Substrats nicht vollständig zu Biogas abgebaut wurde, sondern als mindestens ein lösliches organisches Stoffwechselprodukt mindestens eines Mikroorganismus in der Flüssigphase im Fermentationsprozess angereichert ist. Dieses organische Stoffwechselprodukt ist das gewünschte Zielprodukt. Es ist darüber hinaus möglich auch mehrere verschiedene Stoffwechselprodukte als gewünschte organische Zielprodukte einzuordnen, welche als Gemisch oder separat gewonnen werden sollen. Nachfolgend wird die Erfindung auf Grundlage der Gewinnung eines gewünschten organischen Zielprodukts und von Biogas erläutert. Diese Ausführungen gelten aber ausdrücklich nicht einschränkend, sondern sind auch auf die Gewinnung mehrerer organischer Zielprodukte in einem Fermentationsprozess übertragbar.

Das zugeführte Substrat wird in der Fermentation zu dem gewünschten organischen Zielprodukt und Biogas abgebaut. Die Fermentation kann dabei aus einem Fermenter oder mehreren beliebig verschalteten Fermentern bestehen. Das Zielprodukt wird dabei in mindestens einem der Fermenter angereichert, indem die anaerobe Fermentation durch geeignete Maßnahmen partiell unterdrückt wird. Das gebildete Biogas wird aus der Fermentation zur weiteren Verwendung abgeführt. Aus der Fermentation wird zudem Ablauf abgezogen, so dass der Füllstand des Fermentationssystems im gewünschten Bereich gehalten wird. Der Ablauf enthält das organische Zielprodukt. Ebenso ist es möglich auf diese Weise mehrere Zielprodukte im Ablauf anzureichern.

Der Fermentation werden entweder mit dem organischen Substrat oder durch Dosierung geeigneter Produkte die für den unvollständigen Abbau notwendigen Nährstoffe zugeführt. Dies sind insbesondere Bor, Eisen, Kalium, Kobalt, Kupfer, Magnesium, Mangan, Molybdän, Natrium, Nickel, Phosphor, Schwefel, Selen, Stickstoff, Wolfram und Zink. Je nach eingesetztem Substrat besteht ein unterschiedliches Potenzial zur Anreicherung bestimmter Zielprodukte. Die Anreicherung des gewünschten Zielprodukts bzw. die partielle Unterdrückung der anaeroben Fermentation kann wesentlich durch die Konzentration mindestens eines Nährstoffs beeinflusst werden. Durch Kontrolle der Dosierung mindestens eines relevanten Nährstoffs kann die Konzentration eines Nährstoffes bewusst reduziert oder erhöht werden, um die Bildung eines Zielprodukts zu fördern oder den anaeroben Abbau des angereicherten Zielprodukts durch Limitierung bzw. Inhibierung zu unterdrücken. Darüber hinaus kann durch die Dosierung eines Hemmstoffs die Bildung eines Zielprodukts gefördert oder sein Abbau verhindert werden.

Die Substratzufuhr wird derart gesteuert, dass die gewünschte Anreicherung des Zielprodukts aufrechterhalten wird. Zusätzlich erfolgt die Substratzufuhr derart, dass im Fermenterinhalt ein pH-Wert von 6, vorzugsweise von 7, bevorzugt von 7,5 nicht unterschritten wird. Dadurch wird sichergestellt, dass der Wasserstoffgehalt im Gas kleiner 5%, vorzugsweise kleiner 2%, besonders bevorzugt kleiner 1% ist.

Der Fermentationsprozess wird durch geeignete Maßnahmen in einem günstigen Temperaturbereich durchgeführt. Üblicherweise werden Fermentationen im mesophilen oder thermophilen Bereich betrieben. Durch Variation der Temperatur ist es möglich Einfluss auf die Methangärung und damit auch auf die Zielproduktbildung zu nehmen. Beispielsweise kann eine Temperaturerhöhung um wenige Grad zu einer stärkeren Anreicherung des Zielprodukts führen.

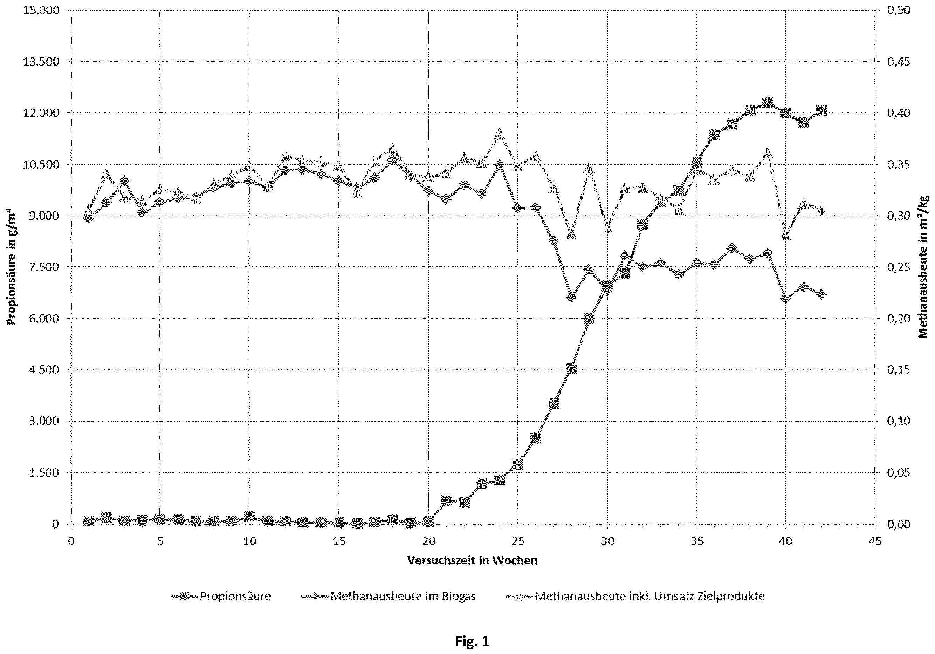

Das Prinzip der Erfindung ist anhand der Versuchsergebnisse zur kontinuierlichen Fermentation zur Anreicherung von Zielprodukten, insbesondere von Propionsäure, und der Bildung von Methan erkennbar. Wesentliche Ergebnisse dieses Versuchs sind Fig. 1 zu entnehmen. In Fig. 1 sind die Wochenmittelwerte des Propionsäuregehalts im Fermentationsmedium sowie der spezifischen Methanausbeuten dargestellt. Letztere sind die spezifischen Methanausbeuten auf Basis des gebildeten Biogases sowie auf Basis des gebildeten Biogases zuzüglich des in den Zielprodukten enthaltenen Gaspotenzials.

Zu Beginn des Versuchs wurde das zugeführte organische Substrat in einem mesophilen anaeroben Fermentationsprozess vollständig zu Biogas bzw. Methan abgebaut. Die spezifische Methanausbeute beträgt im Mittel ungefähr 0,33 m³/kg. Nachfolgend wurde der Gehalt der Nährstoffe im Fermentationsmedium durch eine Reduzierung der Dosierung von Nährstofflösung gezielt abgesenkt, um durch Limitierungen im mikrobiellen Abbauprozess die Konzentration der Zielprodukte im Fermentationsmedium zu erhöhen. Dadurch kam es zu einer partiellen Unterdrückung der anaeroben Fermentation und es war zum Beispiel möglich Propionsäure bis zu einer Konzentration von 12.000 g/m³ im Fermentationsmedium anzureichern. Gleichzeitig wurden durch diesen unvollständigen Substratabbau die Gasausbeute und die Methanausbeute reduziert. Die spezifische Methanausbeute verringerte sich auf etwa 0,25 m³/kg. Unter Berücksichtigung des Methanpotenzials der Zielprodukte ergibt sich jedoch zusammen mit der gemessenen spezifischen Methanausbeute eine vergleichbare spezifische Methanausbeute wie zu Beginn des Versuchs bei vollständigem Substratausbau. Auch während der Anreicherung der Zielprodukte sowie im stationären Bereich am Ende des Versuchs liegt diese berechnete spezifische Methanausbeute bei ungefähr 0,33 m³/kg. Die Anreicherung von Zielprodukten im Fermentationsmedium geht demnach nicht zu Lasten der Substratausnutzung. Darüber hinaus wurde im Versuch festgestellt, dass das Fermentationsgas über den gesamten Versuchszeitraum innerhalb der Messgenauigkeit nur aus den Bestandteilen Methan, Kohlendioxid und Schwefelwasserstoff bestand. Eine verstärkte Bildung von Wasserstoff, wie sie üblicherweise bei der gezielten Bildung von Säuren in einer Hydrolysestufe und/oder Acidogenese auftritt, konnte nicht gemessen werden.

Das im Ablauf enthaltene Zielprodukt kann durch eine Reihe von Prozessen aus dem Ablauf gewonnen und aufgereinigt werden. Je nach Eigenschaft des maßgeblichen Zielprodukts sind diese unterschiedlich gut geeignet. Mögliche Prozesse sind die Destillation, die Extraktion, die Fest-Flüssig-Trennung, Kristallisation, Membrantrennung sowie Strippung. Für den Fachmann selbstverständlich ist auch die Anwendung von beliebigen Kombinationen. Die Gewinnung des Zielprodukts aus dem Ablauf ist üblicherweise dem Fermentationsprozess nachgeschaltet. Es ist aber ebenso möglich die Gewinnung des Zielprodukts beispielsweise bei einer Membrantrennung oder Strippung auch mit dem Fermentationsprozess zu kombinieren. Das Zielprodukt kann je nach Anforderung als Reinsubstanz, Lösung oder Suspension oder allgemein als Stoffgemisch gewonnen werden.

Die im Prozess anfallenden Reststoffe wie zum Beispiel Gärrest oder Prozesswasser sowie Prozessströme mit unerwünschte Inhaltsstoffen können abgeführt oder zumindest anteilig in den Prozess zurückgeführt werden. Dies kann neben einer Ausbeutesteigerung auch zur Beeinflussung der Bildung des Zielprodukts führen. Durch die prozessinterne Verwendung von Prozessströmen insbesondere Prozesswässern kann auch die Verweilzeit und/oder die Konzentration relevanter Inhaltsstoffe im Fermentationsprozess gezielt beeinflusst werden. Dies kann genutzt werden, um die Anreicherung des Zielprodukts zu beeinflussen.

Nachfolgend wird die Erfindung in mehreren Ausführungsbeispielen detailliert dargestellt. Selbstverständlich ist es möglich, mindestens Teile der Ausführungsbeispiele miteinander zu kombinieren. Insbesondere gilt dies für die Gewinnung bei mehreren Zielprodukten sowie bei der Verfahrensführung der Prozessströme.

### Kurzbeschreibung der Zeichnungsfiguren

Fig. 1 zeigt Daten einer Fermentation zur Anreicherung von Propionsäure und der Bildung von Methan;
Fig. 2 zeigt ein Fließschema einer möglichen ersten Ausführungsform gemäß der Erfindung;
Fig. 3 zeigt ein Fließschema einer möglichen zweiten Ausführungsform gemäß der Erfindung;
Fig. 4 zeigt ein Fließschema einer möglichen dritten Ausführungsform gemäß der Erfindung;

### Detaillierte Beschreibung bevorzugter Ausführungsformen

Ausführungsbeispiele der Erfindung sind in den Zeichnungen dargestellt und werden nachfolgend näher beschrieben.

### Ausführungsbeispiel 1

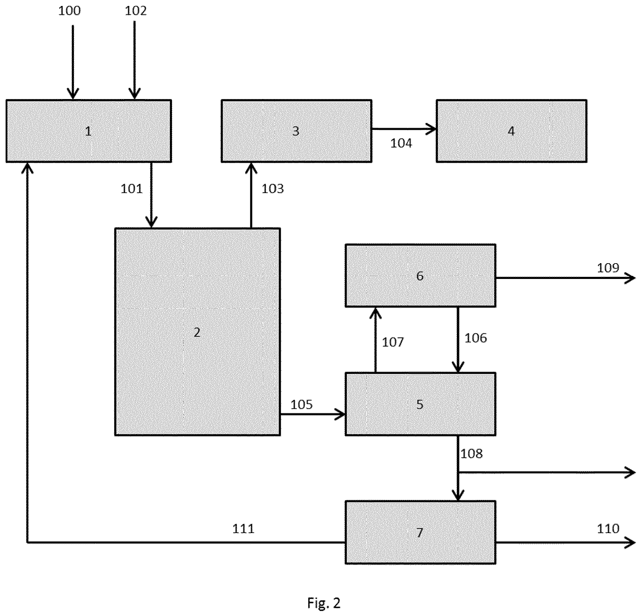

Die Möglichkeit einer technischen Ausführung des Verfahrens sei im Folgenden an der Gewinnung von Propionsäure als Zielprodukt und Biogas veranschaulicht. Propionsäure kann über mikrobielle Abbauwege aus einer Vielzahl von Ausgangsstoffen gebildet werden. Es sind zum Beispiel Abbauwege von Glycerin, Aminosäuren, Glucose und anderen Zuckern wie zum Beispiel Pentosen und auch von organischen Säuren zu Propionsäure bekannt. Selbstverständlich ist auch die Gewinnung von anderen Zielprodukten wie organischen Säuren bzw. mikrobiologischen Stoffwechselprodukten oder deren Mischungen nach dem gleichen Prinzip möglich. Wesentliche Merkmale dieses Ausführungsbeispiels sind auch der Fig. 2 zu entnehmen.

Als organisches Substrat dienen organische Reststoffe aus der Nahrungs- und Genussmittelindustrie wie zum Beispiel Essensreste und Rückstände von Brauereien oder anderen Fermentationsverfahren. Die Verwendung dieser organischen Reststoffe ist wirtschaftlich attraktiv. Je nach Herkunft der organischen Reststoffe können die Inhaltsstoffe variieren. Sie sind durch einen hohen Anteil organischer Reststoffe bzw. NFE (stickstofffreie Extraktstoffe) und im Vergleich zu nachwachsenden Rohstoffen durch einen höheren Anteil an Proteinen gekennzeichnet.

Die Bandbreiten der Zusammensetzung der Trockensubstanz ist nachfolgender Tabelle zu entnehmen:

| Komponente | Gehalt |
|---|---|
| Rohasche | 5% ... 15% |
| Rohfaser | 3% ... 13% |
| Rohprotein | 20% ... 40% |
| Rohfett | 5% ... 20% |
| organischer Rest | 30% ... 60% |

Die organischen Reststoffe (100) werden in einem ersten Prozessschritt (1) zu einem pumpfähigen feststoffhaltigen organischen Substrat (101) homogenisiert und gegebenenfalls temperiert. Dabei kann zur Einstellung eines günstigen Trockensubstanzgehalts Prozesswasser rezirkuliert werden. Das Substrat (101) hat einen organischen Trockensubstanzgehalt von 10% bis 25%.

Das pumpfähige Substrat (101) wird einem kontinuierlichen, anaeroben Fermentationsprozess (2) zugeführt. Die mittlere Belastung der Fermentation (2) mit organischer Substanz wird in einem Bereich von 2 bis 6 kg/m³d eingestellt. Die mittlere Verweilzeit wird in einem Bereich von 20 bis 60 Tagen eingestellt.

Zusätzlich zum Substrat werden dem Fermentationsprozess regelmäßig Nährstoffe zugeführt. Dadurch wird die im Fermenter enthaltene Mikrobiologie gezielt beeinflusst. Durch Realisierung niedriger Gehalte von Kupfer, Selen und Cobalt kommt es zu einer partiellen Unterdrückung der anaeroben Fermentation und in direkter Folge zu einer Anreicherung von Propionsäure im Fermenter. Die Reduzierung der Konzentrationen dieser Stoffe kann durch eine Sulfidfällung im Fermentationsprozess unterstützt werden. Die ausgefällten Verbindungen stehen dann gar nicht mehr oder nur noch sehr eingeschränkt als Nährstoffe für die Mikroorganismen zur Verfügung. Die Gehalte von Kupfer, Selen und Kobalt werden soweit reduziert, dass es zu einer Akkumulation der Propionsäure im Bereich von 5.000 bis 15.000 mg/l im Fermenterinhalt kommt.

Zur Aufrechterhaltung von günstigen Milieubedingungen, insbesondere eines geeigneten pH-Werts, kann die direkte oder indirekte Zuführung von Puffersubstanzen in den Fermentationsprozess sinnvoll sein. Neben dem Einsatz von Substraten mit ausreichender Pufferkapazität kann auch durch eine Rezirkulation von Prozessflüssigkeit der pH-Wert beeinflusst werden. Die Dosierung von Pufferlösungen wie zum Beispiel Natriumhydrogenkarbonat oder Lauge ist prinzipiell auch möglich, jedoch mit zusätzlichen Kosten verbunden. Bei konstanter Fahrweise des Systems und darüber hinaus ausreichender Versorgung mit anderen Nährstoffen ist ein gleichbleibend hoher Umsatz des Substrats unter Bildung von Biogas möglich.

Das Biogas (103) besteht aus Kohlendioxid und Methan sowie Schwefelwasserstoff. Der Gehalt dieser Stoffe, insbesondere der Gehalt an Schwefelwasserstoff variiert je nach Substratzusammensetzung und Prozessbedingungen. Das Vorhandensein von schwefelhaltigen Aminosäuren oder auch Sulfaten im eingesetzten Substrat bzw. in anderen Zuläufen zum Fermentationsprozess führt zu höheren Gehalten von Schwefelwasserstoff im Fermenterinhalt und im Gas. Dessen Gehalt liegt typischerweise in einem Bereich von 0,1% bis 2%. Das gebildete Rohbiogas (103) wird aus dem Fermenter abgeführt und einer Reinigung (3) zugeführt. In dieser Reinigung (3) wird der Schwefelwasserstoff entfernt, so dass das gereinigte Biogas (104) in einem nachgeschalteten BHKW (4) energetisch genutzt werden kann.

Der im Fermentationsprozess mit Propionsäure angereicherte Ablauf (105) wird aus dem Fermenter abgeführt und einer Extraktion (5) zugeführt. Das Extraktionsmittel (106) ist eine Flüssigkeit, welche Propionsäure selektiv anreichert und gleichzeitig wenig bis gar nicht im Wasser gelöst wird. Das Vorhandensein der im Ablauf enthaltenen Feststoffe kann in der Regel bei der Extraktion toleriert werden. Die Extraktion besteht aus einer Abfolge von Mischungs- und Entmischungsprozessen unter Zuführung von frischem, teilbeladenem oder regeneriertem Extraktionsmittel. Dadurch wird ein die Propionsäure enthaltenes Extrakt (107) und ein abgereicherter Ablauf (108) erhalten.

Die Gewinnung der Propionsäure (109) erfolgt in einer thermischen Trennstufe (6), zum Beispiel einer Destillation. Dabei wird das Extraktionsmittel von der Propionsäure getrennt. Während die Propionsäure (109) als Wertstoff zum Beispiel für die chemische Industrie erhalten wird, kann das Extraktionsmittel (106) wieder im Prozess rezirkuliert werden. Auftretende Verluste des Extraktionsmittels müssen dem Prozess wieder zugeführt werden. Die Qualität der gewonnenen Propionsäure ist von der Wahl des Extraktionsmittels und der Ausführung der thermischen Trennstufe abhängig. Die erhaltene Propionsäure kann je nach Anforderung der weiteren Verwendung noch Wasser, Reste des Extraktionsmittels und auch andere Inhaltsstoffe wie zum Beispiel andere organische Säuren enthalten. Zur Verbesserung des Extraktionsprozesses kann ein Ansäuern des Ablaufs (105) hilfreich sein. In diesem Fall wird gebundenes Carbonat als CO2 ausgetrieben und muss abgeführt werden.

Der in der Extraktionsstufe erhaltene abgereicherte Ablauf (108) kann in üblicher Weise genutzt werden. Gegebenenfalls nach einer Fest-Flüssig-Trennung (7) kann die dabei gewonnene flüssige Phase (111) bei der Homogenisierung des Substrats eingesetzt werden. Ein anfallender Feststoff (110) oder aber der abgereicherte Ablauf (108) können auch als Düngemittel verkauft werden.

In einer weiteren Variante kann die Fest-Flüssig-Trennung auch zwischen der Fermentation und der Extraktion erfolgen. In diesem Fall wird aus der Behandlung des Ablaufs ein Feststoff mit Resten von Propionsäure erhalten und nur die erzeugte Flüssigphase, gegebenenfalls nach Absenkung des pH-Werts, der Extraktion zugeführt. Der Säurebedarf zur pH-Einstellung der Flüssigphase kann gegenüber dem Säurebedarf zur pH-Einstellung des Ablaufs deutlich reduziert sein, wenn mit dem Feststoff signifikantes Puffervermögen abgetrennt wird.

### Ausführungsbeispiel 2

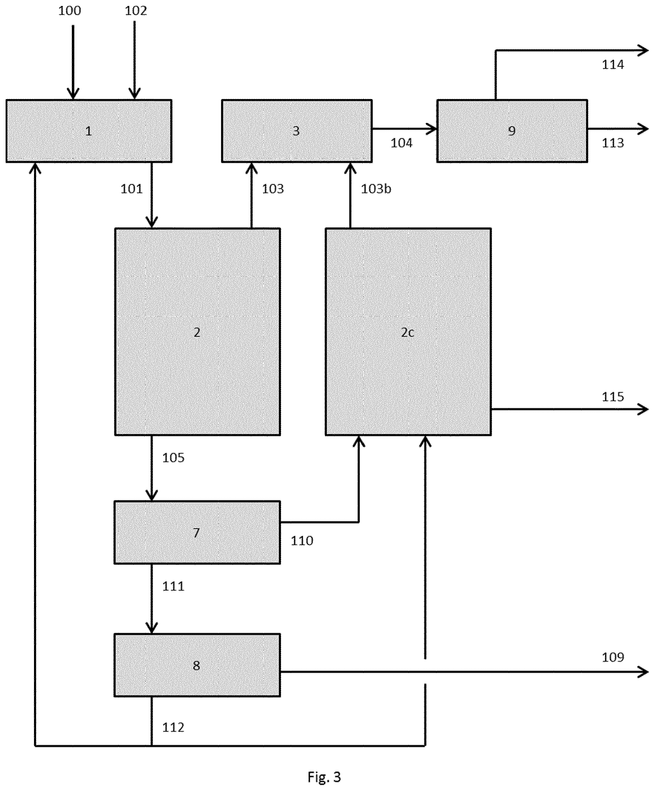

Eine weitere Möglichkeit einer technischen Ausführung des Verfahrens sei im Folgenden anhand der Gewinnung von aromatischen Verbindungen wie zum Beispiel Kresol und/oder Skatol und von Methan veranschaulicht. Diese Substanzen stehen dabei stellvertretend für bei der Fermentation insbesondere von proteinreichen Substraten entstehende Zielprodukte, welche überwiegend undissoziiert vorliegen. Wesentliche Merkmale dieses Ausführungsbeispiels sind der Fig. 3 zu entnehmen. Die nachfolgenden Ausführungen werden beispielhaft nur mit Kresol als Zielprodukt formuliert.

Als organisches Substrat dienen organische Reststoffe wie sie im Ausführungsbeispiel 1 beschrieben sind oder andere organische Substrate mit relevantem Gehalt an Proteinen und/oder aromatischen Verbindungen. Insbesondere organische Substrate mit signifikanten Gehalten der aromatischen Aminosäuren Phenylalanin, Tryptophan oder Tyrosin oder mit Gehalten anderer ähnlicher aromatischer Verbindungen sind für einen solchen Prozess interessant, da mögliche intermediäre Stoffwechselprodukte des anaeroben Abbaus die Zielprodukte Kresol und/oder Skatol sein können. Diese beiden Zielprodukte sind bei längeren Verweilzeiten und ausreichender Nährstoffversorgung vollständig anaerob abbaubar.

Die organischen Reststoffe (100) werden in einem ersten Prozessschritt (1) zu einem pumpfähigen organischen Substrat (101) homogenisiert und gegebenenfalls temperiert. Dabei kann zur Einstellung eines günstigen Trockensubstanzgehalts Prozesswasser rezirkuliert werden. Das Substrat (101) hat einen organischen Trockensubstanzgehalt von 5% bis 30%. Das pumpfähige Substrat (101) wird einem kontinuierlichen, anaeroben Fermentationsprozess (2) zugeführt.

Die mittlere Belastung der Fermentation (2) mit organischer Substanz wird in einem Bereich von 2 bis 8 kg/m³d eingestellt. Die mittlere Verweilzeit wird in einem Bereich von unter 50 Tagen, vorzugsweise unter 30 Tagen, besonders bevorzugt unter 20 Tagen eingestellt. Durch eine hohe Substratzufuhr und vergleichsweise kurze Verweilzeit kommt es zu einer partiellen Unterdrückung der anaeroben Fermentation. In der Fermentation werden zunächst primär leichter abbaubare Substratbestandteile vollständig abgebaut, während sich aromatische Stoffwechselprodukte, insbesondere Kresol, im Fermentationsmedium anreichern.

Die partielle Unterdrückung der anaeroben Fermentation bzw. der weitere anaerobe Abbau des Zielprodukts kann durch hohe Ammoniumgehalte sowie andererseits niedrige Gehalte an Mikronährstoffen beeinflusst werden. Die Nährstoffgehalte können durch regelmäßige Messungen der eingesetzten Substrate und des Fermentationsinhalts sowie eine angepasste Dosierung in günstigen Bereichen gehalten werden. Eine Zuführung von Nährstoffen (102) kann zum Beispiel in die Anmischung (1) erfolgen. Bei konstanter Fahrweise des Systems und darüber hinaus ausreichender Versorgung mit anderen Nährstoffen ist ein gleichbleibend hoher Umsatz des Substrats unter Bildung von Biogas möglich.

Der Kresol enthaltende Ablauf (105) wird aus der Fermentation (2) abgeführt und einer Fest-Flüssig-Trennung (7) zugeführt. Dabei wird der Füllstand und damit der Reaktionsraum in der Fermentation möglichst konstant bzw. zumindest in einem günstigen Bereich gehalten. Die Fest-Flüssig-Trennung (7) erzeugt einen Feststoff (110) und eine Flüssigkeit (111). Letztere enthält den überwiegenden Teil des im Ablauf enthaltenen Kresols. Die Flüssigkeit (111) wird einem Strippprozess (8) zugeführt. In der Strippung (8) wird das wasserdampfflüchtige Kresol mit einem Strippgas aus der Flüssigkeit ausgetrieben und nach Kondensation in wässriger Phase erhalten. Die Strippung kann auch mit anderen Trennoperationen kombiniert ausgeführt werden. Höhere Temperaturen begünstigen den Strippvorgang. Je nach Prozessbedingungen kann die Löslichkeit des Kresols in Wasser auch überschritten sein, so dass ein Kristallisat gewonnen werden kann. Aus der Strippung (8) wird das Zielprodukt Kresol (109) für die weitere Verwendung abgeführt. Als zweites Medium wird die abgereicherte Flüssigkeit (112) aus der Strippung (8) abgeführt.

Die abgereicherte Flüssigkeit (112) wird zu einem Teil genutzt, um durch Rezirkulation in die Fermentation (2) die gewünschte Verweilzeit einzustellen. Dabei kann die abgereicherte Flüssigkeit zunächst in die Anmischung (1) oder alternativ auch direkt in die Fermentation (2) geleitet werden.

Der andere Teil der abgereicherten Flüssigkeit wird zusammen mit dem Feststoff (110) der Nachgärung (2c) zugeführt. In der Nachgärung wird das im Feststoff und der abgereicherten Flüssigkeit enthaltene Restgaspotenzial durch eine kontinuierliche anaerobe Fermentation genutzt.

Das entstehende Rohbiogas der Nachgärung (103b) wird zusammen mit dem Rohbiogas (103) aus der ersten Fermentation (2) einer Reinigung (3) zugeführt. Dort werden Schwefelwasserstoff und gegebenenfalls andere Komponenten abgetrennt. Das gereinigte Biogas (104) wird weiter in die CO2-Entfernung (9) geleitet. Dort wird das Kohlendioxid zum Beispiel durch eine Druckwechseladsorption abgetrennt und abgeführt (113), während das erhaltene Methan (114) für die weitere Verwendung gewonnen wird. Das Methan kann nach Einstellung des Heizwerts zum Beispiel in das Erdgasnetz eingespeist und einer stofflichen oder energetischen Verwertung zugeführt werden.

Der aus der Nachgärung (2c) abgeführte Gärrest (115) enthält die mit dem Substrat und/oder den Nährstoffen zugeführten Mineralstoffe und kann einer stofflichen Verwertung zum Beispiel als Dünger zugeführt werden. In einer alternativen Ausgestaltung kann ein Teil der abgereicherten Flüssigkeit (112) auch zusammen mit dem Gärrest (115) der Nachgärung abgeführt werden. Ebenso ist eine separate Abführung denkbar.

In einer weiteren Ausgestaltung ist es auch möglich das Zielprodukt mit einem geeigneten Strippmedium direkt aus dem Fermentationsprozess zu gewinnen. Möglich ist zum Beispiel das Rezirkulieren von Biogas durch das Fermentationsmedium mit einer Abscheidung des Zielprodukts außerhalb der Fermentation.

### Ausführungsbeispiel 3

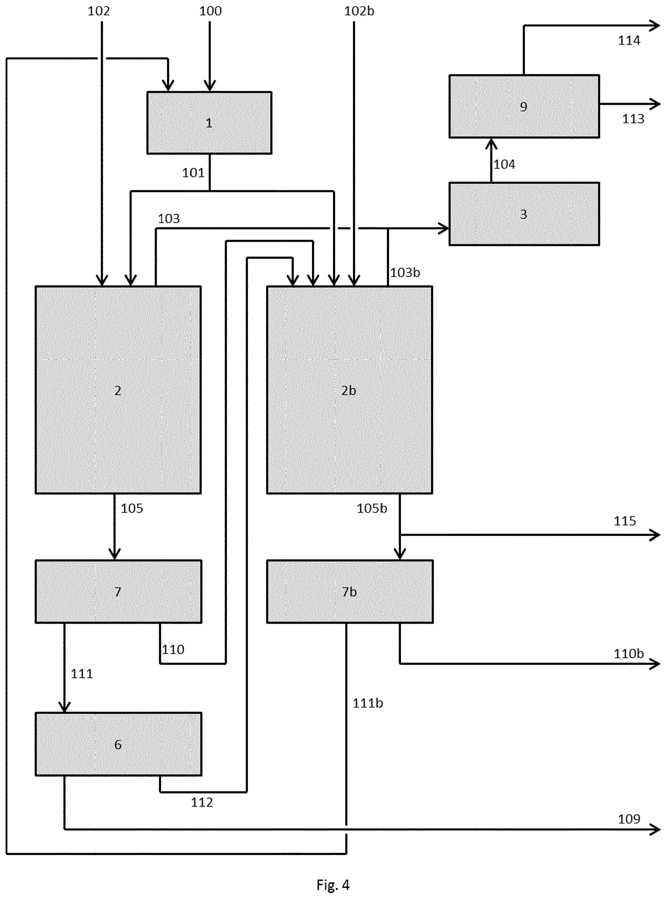

Eine weitere Möglichkeit einer technischen Ausführung des Verfahrens sei im Folgenden anhand der Gewinnung von Isovaleriansäure und von Methan veranschaulicht. Ebenso kann dieses Verfahren auch für die Gewinnung anderer organischer Säuren, insbesondere kurzkettiger oder aromatischer Carbonsäuren wie zum Beispiel Isobuttersäure, Buttersäure, Valeriansäure, Capronsäure und/oder Benzoesäure oder entsprechenden Mischungen angewandt werden. Charakteristisch für diese Zielprodukte ist ihre Anreicherung in dissoziierter Form im Fermentationsmedium. Wesentliche Merkmale dieses Ausführungsbeispiels sind der Fig. 4 zu entnehmen.

Als organisches Substrat dienen organische Reststoffe wie sie im Ausführungsbeispiel 1 beschrieben sind oder andere organische Substrate mit relevantem Gehalt an organischen Verbindungen basierend auf mindestens C5- und/oder C6-Strukturen. Dies können insbesondere Proteine bzw. Aminosäuren sowie Kohlenhydrate wie zum Beispiel Pentosane sein. Derartige Verbindungen können über das Stoffwechselprodukt Isovaleriansäure anaerob abgebaut werden. Durch eine partielle Unterdrückung der anaeroben Fermentation ist es möglich, die Isovaleriansäure im Fermentationsmedium anzureichern. Grundsätzlich sollten im Substrat relevante Mengen an Verbindungen vorhanden sein, welche aus Grundbausteinen mit einer mindestens so hohen Anzahl an verbundenen C-Atomen besteht wie auch C-Atome im Zielprodukt vorhanden sind.

Die organischen Reststoffe (100) werden in einem ersten Prozessschritt (1) zu einem pumpfähigen organischen Substrat (101) homogenisiert und gegebenenfalls temperiert. Dabei kann zur Einstellung eines günstigen Trockensubstanzgehalts Prozesswasser rezirkuliert werden. Das Substrat (101) hat einen organischen Trockensubstanzgehalt von 5% bis 30%. Das pumpfähige Substrat (101) wird anteilig einem anaeroben Fermentationsprozess (2) zugeführt. Ein anderer Teil des Substrats wird einem weiteren anaeroben Fermentationsprozess (2b) zugeführt. Es ist ebenso möglich die Anmischung separat für die jeweiligen Fermentationsprozesse auszuführen, um die Zufuhr der Reststoffe und/oder Prozesswässer in die einzelnen Fermentationen zu steuern.

Während in der einen Fermentation (2) die Prozessbedingungen so eingestellt werden, dass es zu einer partiellen Unterdrückung der anaeroben Fermentation und in der Folge zu einer verminderten Gasbildung (103) und Anreicherung der Isovaleriansäure im Fermentationsmedium kommt, werden in der anderen Fermentation (2b) die Prozessbedingungen so gewählt, dass der vollständige anaerobe Substratabbau nicht eingeschränkt bzw. gefördert wird. Die mittlere Belastung der Fermentationen (2, 2b) mit organischer Substanz wird in einem Bereich von 2 bis 8 kg/m³d eingestellt. Die mittlere Verweilzeit wird in einem Bereich von 20 bis 60 Tagen eingestellt.

Um die anaerobe Fermentation (2) gezielt partiell zu unterdrücken und damit die Anreicherung der Isovaleriansäure im Fermentationsmedium zu begünstigen, wird eine vergleichsweise hohe Substratzufuhr realisiert. Mit dem gleichen Ziel werden zudem eine im Vergleich zur anderen Fermentation (2b) höhere Fermentationstemperatur eingestellt und/oder die Nährstoffversorgung verringert und/oder die Konzentration an Sulfid und/oder Ammonium im Fermentationsmedium bewusst hoch gehalten. Vorteilhaft sind Ammoniumgehalte von mindestens 4.000 mg/l, bevorzugt mindestens 5.000 mg/l, besonders bevorzugt mindestens 6.000 mg/l und ganz besonders bevorzugt mindestens 7.000 mg/l im Fermentationsmedium.

Zur effizienten Ausnutzung des Substrats beim anaeroben Abbau sind die Elemente Bor, Eisen, Kalium, Kobalt, Kupfer, Magnesium, Mangan, Molybdän, Natrium, Nickel, Phosphor, Schwefel, Selen, Stickstoff, Wolfram und Zink von Bedeutung. Je nach Konzentration und Verfügbarkeit der jeweiligen Elemente in den zugeführten Reststoffen und deren Konzentration und Verfügbarkeit im Fermentationsmedium, müssen diese zusätzlich zugeführt werden. Diese Nährstoffe (102, 102b) werden den jeweiligen Fermentationen (2, 2b) getrennt zugeführt.

Zur Aufrechterhaltung eines geeigneten pH-Werts, kann die direkte oder indirekte Zuführung von Puffersubstanzen in den Fermentationsprozess sinnvoll sein. Neben dem Einsatz von Substraten mit ausreichender Pufferkapazität kann auch durch eine Rezirkulation von Prozessflüssigkeit der pH-Wert beeinflusst werden. Die Dosierung von Pufferlösungen wie zum Beispiel Natriumhydrogenkarbonat oder Lauge ist prinzipiell auch möglich, jedoch mit zusätzlichen Kosten verbunden. Die Fermentation wird bei einem pH-Wert des Fermentationsmediums von mindestens 6, vorzugsweise von mindestens 7, bevorzugt von mindestens 7,5 betrieben.

Der Isovaleriansäure enthaltene Ablauf (105) wird aus der Fermentation (2) abgeführt und einer Fest-Flüssig-Trennung (7) zugeführt. Dabei wird der Füllstand und damit der Reaktionsraum in der Fermentation möglichst konstant bzw. zumindest in einem günstigen Bereich gehalten. Die Fest-Flüssig-Trennung (7) erzeugt einen Feststoff (110) und eine Flüssigkeit (111). Letztere enthält den überwiegenden Teil der im Ablauf enthaltenen Isovaleriansäure, da sie überwiegend in dissoziierter Form gelöst vorliegt.

Die Flüssigkeit (111) wird einer thermischen Trennung (6) zugeführt. Bei dieser thermischen Trennung handelt es sich beispielsweise um eine mindestens einstufige Destillation. Durch Absenkung des pH-Werts wird die Valeriansäure in ihre undissoziiierte Form überführt, so dass die Abtrennung verbessert wird. Zur effizienten Abtrennung der Isovaleriansäure wird die Mischungslücke mit Wasser bei höheren Konzentrationen genutzt. Diese Phase kann durch weitere Aufreinigungen, beispielsweise eine weitere Destillation weiter aufgetrennt bzw. fraktioniert werden. Dadurch ist es möglich eine Isovaleriansäurephase mit mehr als 30%, bevorzugt mehr als 40%, besonders bevorzugt mehr als 50% ganz besonders bevorzugt mehr als 60% zu erhalten. Speziell bei der Bildung verschiedener organischer Säuren als Zielprodukte in der Fermentation (2) kann eine solche Fraktionierung nachgeschaltet werden.

Die in der thermischen Trennung anfallende abgereicherte Flüssigkeit (112) wird zusammen mit dem abgetrennten Feststoff (110) der anderen Fermentation (2b) zugeführt. Alternativ kann die abgereicherte Flüssigkeit (112) auch ausgeschleust werden.

In der Fermentation (2b) werden die Bedingungen so eingestellt, dass es zu einer optimalen Gasausnutzung kommt. Dadurch wird nicht nur das Zugeführte Substrat anaerob zu Biogas vergoren, sondern auch das möglicherweise im Feststoff (110) und der abgereicherten Flüssigkeit (112) enthaltene Restgaspotenzial genutzt.

Der aus der Fermentation (2b) abgeführte Ablauf (105b) wird entweder mindestens anteilig direkt als Gärrest (115) ausgeschleust und beispielsweise einer stofflichen Verwertung zugeführt und/oder mindestens anteilig einer Fest-Flüssig-Trennung (7b) zugeführt, um Flüssigkeit (111b) zur Anmischung der Reststoffe oder für andere Nutzungswege zu generieren. Der dabei erzeugte Feststoff (110b) wird in geeigneter Weise abgeführt und einer stofflichen und/oder energetischen Verwertung zugeführt.

Das in den Fermentationen (2, 2b) gebildete Rohbiogas besteht im Wesentlichen jeweils aus den Bestandteilen Methan, Kohlendioxid und Schwefelwasserstoff sowie in Abhängigkeit von der Temperatur und dem Druck von Wasserdampf. Auch bei der Fermentation (2) zur Gewinnung der Isovaleriansäure als Zielprodukt entweicht Rohbiogas (103), welches jedoch keine signifikanten Mengen Wasserstoff enthält. Der Gehalt an Wasserstoff liegt in der Regel unter 1%.

Das entstehende Rohbiogas der Fermentationen (103, 103b) wird zusammen einer Reinigung (3) zugeführt. Dort werden Schwefelwasserstoff und gegebenenfalls andere Komponenten abgetrennt. Das gereinigte Biogas (104) wird weiter in die CO2-Entfernung (9) geleitet. Dort wird das Kohlendioxid zum Beispiel durch eine Druckwechselabsorption abgetrennt und abgeführt (113), während das erhaltene Methan (114) für die weitere Verwendung gewonnen wird. Das Methan kann nach Einstellung des Heizwerts zum Beispiel in das Erdgasnetz eingespeist und einer stofflichen oder energetischen Verwertung zugeführt werden.

### Bezugszeichenliste

- 100: Reststoffe
- 101: Substrat
- 102: Nährstoffe
- 102b: Nährstoffe
- 103: Rohbiogas
- 103b: Rohbiogas
- 104: Biogas
- 105: Ablauf
- 105b: Ablauf
- 106: Extraktionsmittel
- 107: Extrakt
- 108: abgereicherter Ablauf
- 109: Zielprodukt
- 110: Feststoff
- 110b: Feststoff
- 111: Flüssigkeit
- 111b: Flüssigkeit
- 112: abgereicherte Flüssigkeit
- 113: CO₂
- 114: Methan
- 115: Gärrest
- 1: Anmischung
- 2: Fermentation
- 2b: Fermentation
- 2c: Nachgärung
- 3: Reinigung
- 4: BHKW
- 5: Extraktion
- 6: thermische Trennung
- 7: Fest-Flüssig-Trennung
- 7b: Fest-Flüssig-Trennung
- 8: Strippung
- 9: CO2-Entfernung

## Patentansprüche

1. Verfahren zur Gewinnung mindestens eines organischen Zielprodukts und von Biogas, **gekennzeichnet dadurch, dass**
a) heterogenes organisches Substrat einem anaeroben Fermentationsprozess zugeführt wird;
b) bei diesem anaeroben Fermentationsprozess eine Hydrolyse, Versäuerung und Methanisierung durch eine Mischkultur aus Bakterien und Archaeen in einem Reaktionsraum durchgeführt werden;
c) eine partielle Unterdrückung der anaeroben Fermentation durch die hydraulische Verweilzeit des anaeroben Fermentationsprozesses und/oder die Substratzufuhr und/oder die Temperatur des anaeroben Fermentationsprozesses und/oder die Limitierung mindestens eines Nährstoffs im anaeroben Fermentationsprozess und/oder die Konzentration eines Hemmstoffes im anaeroben Fermentationsprozess gesteuert wird, wodurch ein Teil des organischen Substrats nicht vollständig zu Biogas bzw. Methan abgebaut wird;
d) mindestens ein Zielprodukt als organisches Stoffwechselprodukt einer Mischkultur aus Bakterien im Fermentationsprozess durch den gezielten unvollständigen Abbau des organischen Substrats angereichert wird;
e) das im anaeroben Fermentationsprozess durch die Archaeen gebildete Biogas einen Anteil von Wasserstoff von kleiner 5% aufweist und zur weiteren Verwendung gewonnen wird; und
f) mindestens ein organisches Zielprodukt zur weiteren Verwendung aus dem anaeroben Fermentationsprozess gewonnen wird.

2. Verfahren nach Anspruch 1, **gekennzeichnet dadurch, dass** der Anteil an Wasserstoff des beim anaeroben Fermentationsprozess gebildeten Biogases kleiner 2%, besonders bevorzugt kleiner 1% ist.

3. Verfahren nach einem der vorherigen Ansprüche, **gekennzeichnet dadurch, dass** aus dem anaeroben Fermentationsprozess mindestens zwei Zielprodukte separat gewonnen werden.

4. Verfahren nach einem der vorherigen Ansprüche, **gekennzeichnet dadurch, dass** ein bei der Gewinnung eines Zielprodukts anfallender Stoffstrom wieder einem anaeroben Fermentationsprozess zugeführt wird.

5. Verfahren nach einem der vorherigen Ansprüche, **gekennzeichnet dadurch, dass** die Gewinnung eines Zielprodukts nach einer Fest-Flüssig-Trennung des Fermentationsmediums erfolgt.

6. Verfahren nach einem der vorherigen Ansprüche, **gekennzeichnet dadurch, dass** der anaerobe Fermentationsprozess mehrstufig durchgeführt wird.

7. Verfahren nach einem der vorherigen Ansprüche, **gekennzeichnet dadurch, dass** der anaerobe Fermentationsprozess bei einem pH-Wert größer 6, vorzugsweise größer 7, bevorzugt größer 7,5 realisiert wird.

## Claims

1. A method for the production of at least one organic target product and of biogas, **characterized in that**:
a) heterogeneous organic substrate is supplied to an anaerobic fermentation process;
b) hydrolysis, acidification and methanation are carried out in a reaction chamber during this anaerobic fermentation process using a mixed culture of bacteria and archaea;
c) partial suppression of the anaerobic fermentation is controlled by the hydraulic residence time for the anaerobic fermentation process and/or the substrate supply and/or the temperature of the anaerobic fermentation process and/or by limiting at least one nutrient in the anaerobic fermentation process and/or the concentration of an inhibitor in the anaerobic fermentation process, whereupon a portion of the organic substrate is not completely decomposed into biogas or methane;
d) at least one target product is enriched as an organic metabolite of a mixed culture of bacteria in the fermentation process by the deliberately incomplete decomposition of the organic substrate;
e) the biogas formed by the archaea in the anaerobic fermentation process has a hydrogen content of less than 5% and is recovered for further use; and
f) at least one organic target product is produced from the anaerobic fermentation process for further use.

2. The method of claim 1, **characterized in that** the fraction of hydrogen in the biogas formed during the anaerobic fermentation process is less than 2%, particularly preferably less than 1%.

3. The method of one of the preceding claims, **characterized in that** at least two target products are obtained separately from the anaerobic fermentation process.

4. The method of one of the preceding claims, **characterized in that** a substance stream arising during the production of a target product is reintroduced into an anaerobic fermentation process.

5. The method of one of the preceding claims, **characterized in that** a target product is produced following a solid-liquid separation of the fermentation medium.

6. The method of one of the preceding claims, **characterized in that** the anaerobic fermentation process is carried out in multiple stages.

7. The method of one of the preceding claims, **characterized in that** the anaerobic fermentation process is conducted with a pH value of greater than 6, preferably greater than 7, more preferably greater than 7.5.

## Revendications

1. Procédé permettant d'obtenir au moins un produit cible organique et du biogaz, **caractérisé en ce que** l'on
a) alimente un processus de fermentation anaérobie en substrat organique hétérogène ;
b) réalise, dans le cadre dudit processus de fermentation anaérobie, une hydrolyse, une acidification et une méthanisation au moyen d'une culture mixte de bactéries et d'archées au sein d'une chambre de réaction ;
c) pilote une suppression partielle de la fermentation anaérobie en jouant sur la durée de séjour hydraulique du processus de fermentation anaérobie et/ou l'alimentation en substrat et/ou la température du processus de fermentation anaérobie et/ou la limitation d'au moins en élément nutritif au sein du processus de fermentation anaérobie et/ou la concentration d'un agent inhibiteur au sein du processus de fermentation anaérobie, pour faire en sorte qu'une partie du substrat organique ne soit pas complètement dégradée en biogaz ou méthane ;
d) on provoque, de par cette dégradation partielle ciblée du substrat organique, l'accumulation d'au moins un produit cible en tant que produit métabolique organique d'une culture mixte de bactéries dans le cadre dudit processus de fermentation ;
e) obtient du biogaz qui, suite à la formation de ce dernier par les archées dans le cadre du processus de fermentation anaérobie, contient une proportion d'hydrogène inférieure à 5 % et qui est destiné à une utilisation en aval ; et
f) obtient, à partir du processus de formation anaérobie, au moins un produit cible organique qui est destiné à une utilisation en aval.

2. Procédé selon la revendication 1, **caractérisé en ce que**
le biogaz formé lors du processus de fermentation anaérobie comporte une proportion d'hydrogène inférieure à 2 %, avec une préférence particulière inférieure à 1 %.

3. Procédé selon l'une des revendications précédentes, **caractérisé en ce que** l'on obtient, à partir du processus de fermentation anaérobie, au moins deux produits cibles séparément.

4. Procédé selon l'une des revendications précédentes, **caractérisé en ce qu'**un flux obtenu lors de l'obtention d'un produit cible est recyclé dans le processus de fermentation anaérobie.

5. Procédé selon l'une des revendications précédentes, **caractérisé en ce que** le produit cible est obtenu après avoir soumis le milieu de fermentation à une séparation solide/liquide.

6. Procédé selon l'une des revendications précédentes, **caractérisé en ce que** le processus de fermentation anaérobie est réalisé en plusieurs étapes.

7. Procédé selon l'une des revendications précédentes, **caractérisé en ce que** le processus de fermentation anaérobie est réalisé à un pH supérieur à 6, de préférence supérieur à 7, préférentiellement supérieur à 7,5.
